# EUROPÄISCHE PATENTSCHRIFT

(11) **EP 2 459 240 B1**
(45) Veröffentlichungstag und Bekanntmachung des Hinweises auf die Patenterteilung: **11.03.2015**
(21) Anmeldenummer: 10737263.3
(22) Anmeldetag: 06.07.2010
(51) Int. Cl.: A61L 27/30, A61L 27/50

(54) **STRUKTURIERTE OBERFLÄCHEN FÜR IMPLANTATE**
STRUCTURED SURFACES FOR IMPLANTS
SURFACES STRUCTURÉES POUR IMPLANTS

(30) Priorität: 31.07.2009 DE 102009035795
(43) Veröffentlichungstag der Anmeldung: 06.06.2012
(73) Patentinhaber: Leibniz-Institut für Neue Materialien gemeinnützige GmbH, 66123 Saarbrücken (DE)
(72) Erfinder: VEITH, Michael, 66386 St.-Ingbert (DE); AKTAS, Oral Cenk, 66123 Saarbrücken (DE); OBERRINGER, Martin, 66539 Neunkirchen (DE); METZGER, Wolfgang, 66121 Saarbrücken (DE)
(74) Vertreter: Patentanwälte Gierlich & Pischitzis Partnerschaft
(86) Internationale Anmeldenummer: PCT/EP2010/004075
(87) Internationale Veröffentlichungsnummer: WO 2011/012213

(56) Entgegenhaltungen:
- EP-A2- 1 679 088
- WO-A1-02/03894
- WO-A1-2009/034197
- WO-A2-2006/118595
- DE-A1-102007 053 023

## Beschreibung

### Gebiet der Erfindung

Die Erfindung betrifft die Verwendung einer strukturierten Beschichtung für Implantate, insbesondere für Endoprothesen, sowie ein Verfahren zur Herstellung einer solchen Beschichtung.

### Stand der Technik

Ein immer noch ungelöstes Problem bei Implantaten, insbesondere von Endoprothesen, ist die aseptische Prothesenlockerung. Diese bewirkt, dass ca. 10 % aller Implantate innerhalb der ersten 15 Jahre neu untersucht oder justiert werden müssen.

Diese Komplikation beginnt normalerweise mit einer reduzierten Anhaftung von Osteoblasten an der Implantatoberfläche. Dies führt zu Abkapselung des Implantats, oft verbunden mit der Ausbildung eines mit Flüssigkeit gefüllten Hohlraums. Dies begünstigt die Prothesenlockerung, da noch zusätzlich die Regeneration des Gewebes behindert wird. Dabei wird die Abkapselung durch die Anhaftung von Fibroblasten an der Implantatoberfläche begünstigt.

Im Gegensatz dazu beschreibt die sogenannte Osseointegration die ideale Reaktion des umgebenden Knochens mit dem Implantat, d. h. eine direkte Verbindung des neuen gebildeten Knochens mit der Oberfläche des Implantats ohne eine weiche Gewebeschicht.

Als Materialien für klinische Anwendungen werden Titan, Titanlegierungen, aber auch keramische Materialien, wie Aluminiumoxid oder Zirkoniumoxid. Dabei weist insbesondere Aluminiumoxid eine gute Biokompatibilität auf.

Allerdings kommt es neben dem Material der Oberfläche entscheidend auf die Oberflächenstruktur des Implantats an. Dabei ist bekannt, dass Strukturen im Bereich von Mikrometern, aber auch im Bereich von Nanometern die Haftung von Osteoblasten gegenüber der Anhaftung von Fibroblasten begünstigen. Dies wird auch maßgeblich durch das verwendete Material, sowie seine Oberflächentopographie sowie seine Rauheit und Porosität bestimmt. Dabei ist eine Strukturierung im Bereich von Mikrometern vorteilhaft (Jager M, Zilkens C, Zanger K, Krauspe R. Significance of nano- and microtopography for cell-surface interactions in orthopaedic implants. J Biomed Biotechnol 2007;2007(8):69036; Hulbert SF, Young FA, Mathews RS, Klawitter JJ, Talbert CD, Stelling FH. Potential of ceramic materials as permanently implantable skeletal prostheses. J Biomed Mater Res 1970;4 (3) :433-456) .

In mehreren Arbeiten wurde eine gute Kompatibilität von Aluminiumoxid, insbesondere von nanoporösen Membranen mit einem Porendurchmesser von 30-80 nm gezeigt (Webster T, Hellenmeyer E, Price R. Increased osteoblast functions on theta plus delta nanofiber alumina. Biomaterials 2005;26(9):953-960; Swan E, Popat K, Grimes C, Desai T. Fabrication and evaluation of nanoporous alumina membranes for osteoblast culture. Journal of Biomedical Materials Research Part A 2005;72A(3):288-295).

Aus der Schrift WO 2008/011920 A1 der Anmelderin sind Nanodrähte, bzw. eine eindimensionale Kompositstruktur bestehend aus einem metallischen Kern und ummantelt mit einem Metalloxid, insbesondere aus Aluminium und Aluminiumoxid bekannt.

Die Patentanmeldung WO 2006/118595 A offenbart nanostrukturierte Beschichtungen enthaltend ein isolierendes anorganisches Material wie z.B. Al₂O₃. Diese Beschichtungen können für medizinische Artikel wie Implantate verwendet werden.

EP 1679088 A offenbart medizinische Implantate mit einer Beschichtung aus nanostrukturiertem Material (z.B. Aluminiumoxid).

WO 02/03894 A offenbart eine Prothese mit einer porösen Beschichtung aus Aluminiumoxid, wobei die Porengrösse 5 bis 200 Nanometer beträgt und die Poren mit einem bioaktiven Material gefüllt sind.

DE 102007053023 A offenbart ein Verfahren zur Herstellung von Oxidverbindungen als Beschichtungszusammensetzung, bei dem durch Thermolyse von z.B. Aluminum-tert-butoxydihydrid or Gallium-tert-butoxydihydrid bei >400°C eine Element/Elementoxid-Kompositstruktur gebildet wird, in der der Oxidanteil durch Erhitzen erhöht wird.

Allerdings sind das ideale Material und die ideale Oberflächenstruktur noch lange nicht gefunden. Auch zeigen die Studien oft widersprechende Ergebnisse bezüglich der benötigten Eigenschaften der Oberfläche. So bestimmt auch der Zelltyp die Kompatibilität maßgeblich mit. Es ist daher auch von hoher Bedeutung, dass die Struktur einer Oberfläche auf einfache Weise strukturiert und angepasst werden kann.

### Aufgabe

Aufgabe der Erfindung ist es, eine strukturierte Beschichtung bereitzustellen, welche die beschriebenen Nachteile des Standes der Technik löst und sich zur Verwendung als Beschichtung von Implantaten, insbesondere Endoprothesen, eignet. Außerdem soll ein Verfahren zur Herstellung solcher Beschichtungen bereitgestellt werden.

### Lösung

Diese Aufgabe wird durch die Erfindungen mit den Merkmalen von Anspruch 1 gelöst. Vorteilhafte Weiterbildungen der Erfindungen sind in den Unteransprüchen gekennzeichnet.

Die Aufgabe wird gelöst durch die Verwendung einer strukturierten Beschichtung zur Beschichtung von Implantaten, wobei die strukturierte Beschichtung mindestens aus einem Oxid ausgewählt aus Aluminiumoxid, Galliumoxid, Indiumoxid und Thalliumoxid besteht und eine Mikrostruktur und/oder eine Nanostruktur , bevorzugt aus Aluminiumoxid, aufweist und die Beschichtung durch Bestrahlung einer eindimensionalen Kompositstruktur erhalten wurde.

Dabei wird unter eine Mikrostruktur eine Struktur verstanden, welche mindestens eine Dimension aufweist, die kleiner als ein Millimeter aber größer als ein Mikrometer ist. Dementsprechend weisen Nanostrukturen mindestens eine Dimension auf, welche kleiner als ein Mikrometer ist.

Die Strukturierung ist insbesondere auf der Oberfläche der Beschichtung angeordnet.

Es wurde überraschenderweise gefunden, dass eine solche Beschichtung besonders vorteilhafte Eigenschaften zur Verwendung in Implantaten aufweist. So ist die Anhaftung von Fibroblasten deutlich reduziert.

Mit Vorteil weist die Beschichtung eine Mikrostruktur im Bereich (Größenordnung) von 1 bis 100 µm auf, bevorzugt zwischen 1 und 10 µm, besonders bevorzugt zwischen 1 und 6 µm, insbesondere zwischen 3 und 5 µm.

Die erfindungsgemäße Beschichtung kann auch das Metall und das entsprechende Metalloxid umfassen. Das Metalloxid bildet dabei mindestens die Oberfläche der Beschichtung, während das Metall nicht an der Oberfläche angeordnet ist.

Die Beschichtung umfasst eine eindimensionale Kompositstruktur aus einem Metall und einem Metalloxid, wobei das Metall ausgewählt ist aus der Gruppe enthaltend A1, Ga, In oder Tl. Dabei ist eine eindimensionale Kompositstruktur ein Komposit aus einem metallischen Kern und einer Metalloxid-Hülle. Die eindimensionale Kompositstruktur kann einen oder mehrere Nanodrähte des beschriebenen Aufbaus umfassen bzw. daraus bestehen. Neben diesen einfachen, linearen, kabelartigen, eindimensionalen Strukturen kann die eindimensionale Kompositstruktur alternativ oder zusätzlich eine oder mehrere verzweigte Strukturen umfassen bzw. daraus bestehen, die aus mehreren, astartig aufeinander aufgewachsenen Nanodrähten der linearen Form aufgebaut sind. Diese beiden Formen können auch als lineare bzw. verzweigte Nanodrähte bezeichnet werden. Bei der verzweigten Form können die metallischen Kerne der Drähte sich an den Verzweigungen berühren oder die Metallkerne können an den Verzweigungen durch die Metalloxidhülle voneinander getrennt sein. Die eindimensionale Kompositstruktur kann frei vorliegen oder sich auf einem Substrat befinden. Bevorzugt ist eine eindimensionale Kompositstruktur aus Aluminium und Aluminiumoxid.

Die Nanodrähte besitzen insbesondere zwei Dimensionen, die im Bereich unterhalb von 200 nm liegen, z.B. im Bereich von 1 bis 200 nm und bevorzugt von 10 bis 100 nm, insbesondere etwa 20 bis 40 nm. Das Verhältnis von Breite zu Länge der Nanodrähte ist im Allgemeinen mindestens 1:3 und bevorzugt mindestens 1:5. Die dritte Dimension liegt in der Regel im Mikrometer und Submikrometerbereich. Der Querschnitt der Nanodrähte ist in der Regel annähernd kreisförmig.

Bevorzugt sind Nanodrähte, wie bereits aus WO 2008011920 A1 bekannt.

In einer Ausführungsform weist die Beschichtung einen Kontaktwinkel (oder Benetzungswinkel) von unter 40°, bevorzugt unter 20° mit Wasser als Messflüssigkeit auf.

In einer weiteren Ausführungsform weist die Beschichtung eine quadratische Rauheit von über 5 µm bevorzugt zwischen 5 µm und 10 µm auf Die strukturierte Beschichtung wird durch Bestrahlung der eindimensionalen Kompositstruktur erhalten, bevorzugt durch Bestrahlung mit einem Laser. Die eindimensionale Kompositstruktur ist bevorzugt ein Breitbandabsorber und kann folglich Licht aus einem breiten Wellenlängenbereich absorbieren. Die Wellenlänge des Lasers kann im Bereich von UV bis zu elektromagnetischen Wellen liegen, vorzugsweise im Bereich von 300 nm bis 15 µm, besonders bevorzugt im Bereich von 500 nm bis 11 µm, noch vorteilhafter, aber nicht beschränkt auf Laser mit den Wellenlängen 488 nm, 514 nm, 532 nm, 635 nm, 1064 nm oder 10.6 µm. Bevorzugt sind Wellenlängen aus einem Bereich von 500 nm bis 700 nm, bevorzugt zwischen 500 nm und 600 nm. Es können kontinuierliche (CW) oder gepulste Laser verwendet werden. Bevorzugt werden gepulste Laser verwendet.

Bei der Bestrahlung kann das Metall der eindimensionale Kompositstruktur vollständig in das Metalloxid überführt werden.

Vorzugsweise liegt die verwendete Laserenergie abhängig von der verwendeten Wellenlänge und der Element/Elementoxid-Kompositstruktur zwischen 1 Milliwatt pro Quadratzentimeter und mehreren Watt pro Quadratzentimeter, bevorzugt zwischen 1 Milliwatt pro Quadratzentimeter und 10 Watt pro Quadratzentimeter, besonders bevorzugt zwischen 1 mW/cm² und 5 W/cm².

Durch die Bestrahlung wird die eindimensionale Kompositstruktur lokal erhitzt. Dadurch kommt es nicht nur zu einer Oxidation des Metalls, insbesondere, wenn die Bestrahlung unter der Anwesenheit von Sauerstoff, z. B. unter Raumluft, durchgeführt wird, sondern auch zu einem lokalen Schmelzen der eindimensionalen Kompositstruktur. Beim Erstarren löst sich die nanoskalige Struktur der Kompositstruktur zumindest teilweise auf und es bilden sich größere Strukturen in der Größenordnung von Mikrometern, beispielsweise zwischen 1 bis 5 µm. Gesteuert durch die Intensität des Lasers und/oder die Häufigkeit und/oder Länge der Laserpulse können auf diese Weise höchst unterschiedlich veränderte Oberflächenstrukturen erzeugt werden, wie z. B. Strukturen, welche sowohl Nanostrukturen, als auch Mikrostrukturen aufweisen. Auf diese Weise kann die Beschichtung auf einfache Weise angepasst werden.

So wurde gefunden, dass sowohl eindimensionale Kompositstrukturen, als auch bestrahlte eindimensionale Kompositstrukturen eine deutlich geringere Adhäsion von Fibroblasten ausweisen, insbesondere bei Bestrahlung mit höherer Intensität, bzw. mehreren Laserpulsen. So scheint die Adhäsion von Fibroblasten sowohl bei der Nanostruktur der eindimensionalen Kompositstruktur, als auch von Mikrostruktur der Oberfläche nach Bestrahlung reduziert zu sein. Dies ist auch daran zu erkennen, dass Fibroblasten keine oder nur wenige Filopodien zur Adhäsion ausbilden und eine stark reduzierte Zelldichte bei Kultivierung auf diesen Beschichtungen gemessen wird. Auch zeigt sich eine starke Abnahme der Proliferation, bzw. der Anzahl der mitotischen Zellen, insbesondere bei den stärker bestrahlten Beschichtungen.

Gleichzeitig konnten bei der Verwendung von Aluminiumoxid auch bei Verwendung der eindimensionalen Kompositstruktur, welche auch reines Aluminium enthält, keine Al³⁺-Ionen in dem Medium der Zellen nachgewiesen werden. Dies zeigt die gute Bioverträglichkeit solcher Beschichtungen.

Außerdem kann diese Wirkung der Bestrahlung der eindimensionalen Kompositstruktur auf die Oberfläche beschränkt werden. So kann die Eindringtiefe des Lasers beispielsweise bei Verwendung eines gepulsten Lasers auf einen Bereich von kleiner ca. 400 nm reduziert werden, bevorzugt kleiner ca. 300 nm, besonders bevorzugt kleiner ca. 200 nm. Dies ermöglicht nicht nur die Herstellung sehr dünner Schichten, sondern auch eine besondere Schonung des Substrats, sowie den Erhalt der Nanostruktur unterhalb der Oberfläche und beispielsweise innerhalb von Poren, welche auch mehrere Mikrometer groß sein können.

Die erfindungsgemäße Beschichtung kann zur Beschichtung unterschiedlichster Implantate und Endoprothese verwendet werden. So erlaubt die Erfindung, insbesondere durch die einfache Steuerung der Oberflächentopographie der Beschichtung eine einfache Anpassung an beliebige Bedingungen. Möglich sind beispielsweise Zahnimplantate, Teile für künstliche Gelenke oder Knochenschrauben.

Insbesondere wird die Kompositstruktur unter Ausbildung von Mikro- und/oder Nanostrukturen bestrahlt.

In einer bevorzugten Ausführungsform wird die Kompositstruktur mit einer gepulsten Lichtquelle bestrahlt, insbesondere mit einem gepulsten Laser. Dabei kann ein solcher Puls beispielswiese zwischen 1 bis 100 ns lang sein, bevorzugt unter 50 ns, besonders bevorzugt unter 20 ns oder 10 ns. Die Pulsfrequenz liegt zwischen 1 Hz und 100 Hz, bevorzugt zwischen 5 Hz und 50 Hz.

Die Anzahl der Pulse liegt bevorzugt bei unter 10, besonders bevorzugt bei unter 7 Pulsen, besonders bevorzugt zwischen 3 und 5 Pulsen.

Abhängig von der Fluenz der Bestrahlung kann eine unterschiedliche Anzahl von Pulsen nötig sein, um eine bestimmte Struktur der Oberfläche zu erzeugen. Die Fluenz kann zwischen 0.01 J/cm² und 2 J/cm² liegen, bevorzugt zwischen 0.1 J/cm² und 0.5 J/cm², besonders bevorzugt zwischen 0.1 und 0.3 J/cm².

Insbesondere bei einer Bestrahlung mit mehr als 2 Pulsen verändert sich die Oberflächentopographie der Beschichtung sehr stark. Die eindimensionale Kompositstruktur schmilzt und wahrscheinlich durch die Ablationsdruck bilden sich sphärische Ausbuchten auf der Oberfläche. Durch die Intensität des Lasers kann die Dichte und Anordnung dieser Strukturen gesteuert werden.

Die Beschichtung der Erfindung kann auch noch weitere Merkmale ausweisen, um die Eigenschaften weiter zu verbessern. So könnten beispielsweise weitere Beschichtungen zur Erhöhung der Biokompatibilität aufgebracht werden. Auch können Wachstumsfaktoren zur Begünstigung des Knochenwachstums an die Oberfläche gebunden oder absorbiert werden, z.B. *bone morphogenic protein I.*

Weitere Einzelheiten und Merkmale ergeben sich aus der nachfolgenden Beschreibung von bevorzugten Ausführungsbeispielen in Verbindung mit den Unteransprüchen.

Die Ausführungsbeispiele sind in den Figuren schematisch dargestellt. Im Einzelnen zeigt:
- Fig. 1: Kontaktwinkel einer eindimensionalen Kompositstruktur aus Al/Al₂O₃ ohne Bestrahlung (a), nach Bestrahlung mit einem Laserpuls (b), mit zwei Laserpulsen (c) und mit drei Laserpulsen (d); die REM-Aufnahmen (REM: Rasterelektronenmikroskop) zeigen die jeweiligen Oberflächen ohne Bestrahlung (a, Maßstab: 1.3 µm), nach Bestrahlung mit einem Laserpuls (b, Maßstab 1.3 µm), mit zwei Laserpulsen (c, Maßstab 2.5 µm) und mit drei Laserpulsen (d, Maßstab 2.5 um);
- Fig. 2: XPS-Spektrum einer eindimensionalen Kompositstruktur aus Al/Al₂O₃ nach Bestrahlung mit einem Laserpuls;
- Fig. 3: Mikroaufnahme von NHDF jeweils mit Antikörpern für CD90 eingefärbt; NHDF auf Glassubstrat (a); NHDF auf einer eindimensionalen Kompositstruktur aus Al/Al₂O₃ ohne Bestrahlung (b); nach einer Bestrahlung mit einem Laserpuls (c); mit zwei Laserpulsen (d); mit drei Laserpulsen (e);
- Fig. 4: REM-Aufnahme von NHDF kultiviert auf Standard-Objektträgern aus Glas;
- Fig. 5: REM-Aufnahmen von NHDF kultiviert auf einer eindimensionalen Kompositstruktur aus Al/Al₂O₃ ohne Bestrahlung;
- Fig. 6: REM-Aufnahme von NHDF kultiviert auf einer eindimensionalen Kompositstruktur aus Al/Al₂O₃ nach Bestrahlung mit einem Laserpuls;
- Fig. 7: REM-Aufnahme von NHDF kultiviert auf einer eindimensionalen Kompositstruktur aus Al/Al₂O₃ nach Bestrahlung mit zwei Laserpulsen; und
- Fig. 8: REM-Aufnahme von NHDF kultiviert auf einer eindimensionalen Kompositstruktur aus Al/Al₂O₃ nach Bestrahlung mit drei Laserpulsen.
- Fig. 9: REM-Aufnahmen von NHDF kultiviert auf einer eindimensionalen Kompositstruktur aus Al/Al₂O₃ nach Bestrahlung mit drei Laserpulsen.

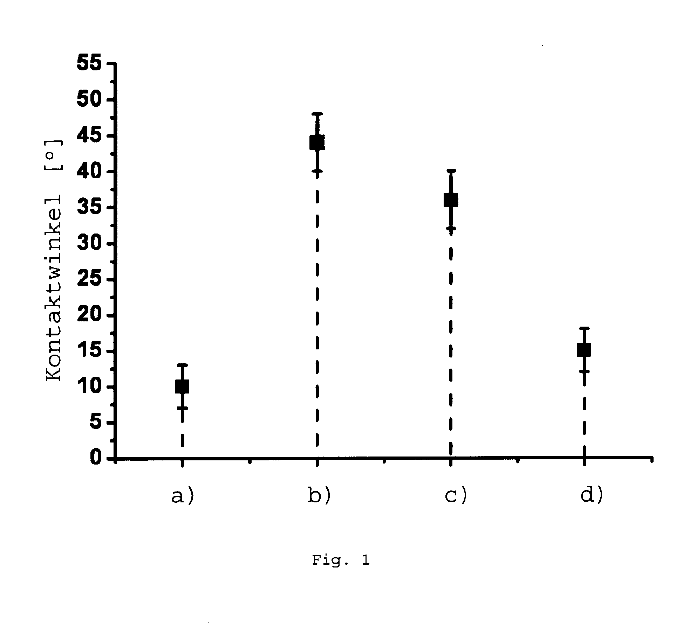

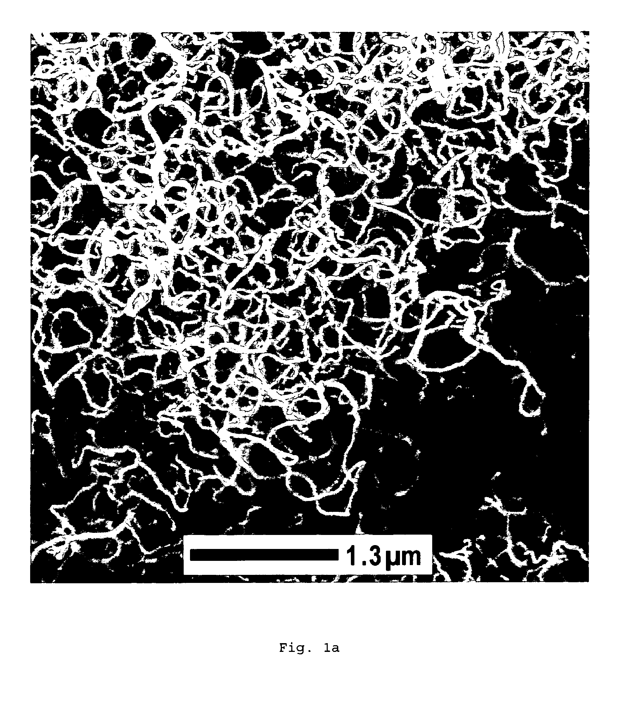

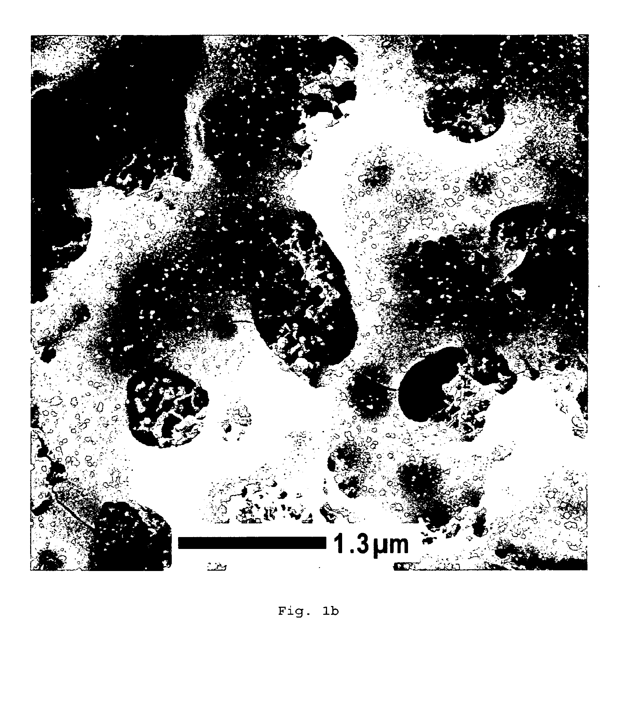

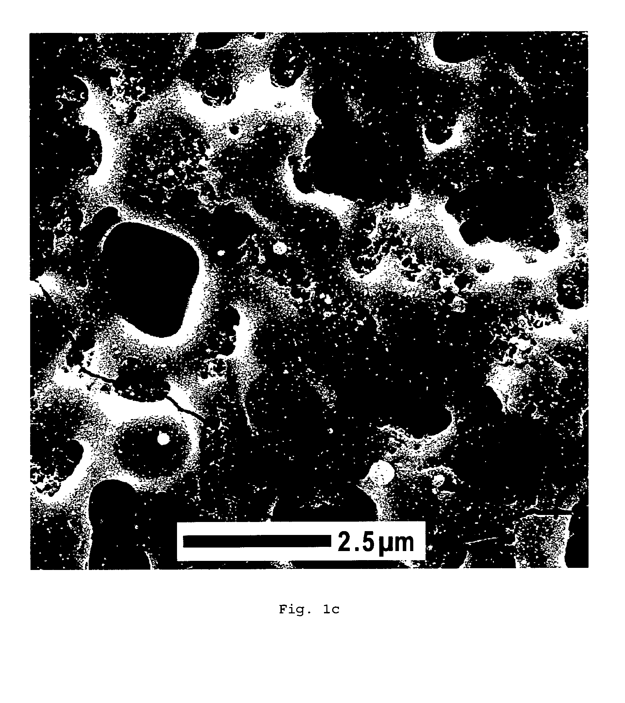

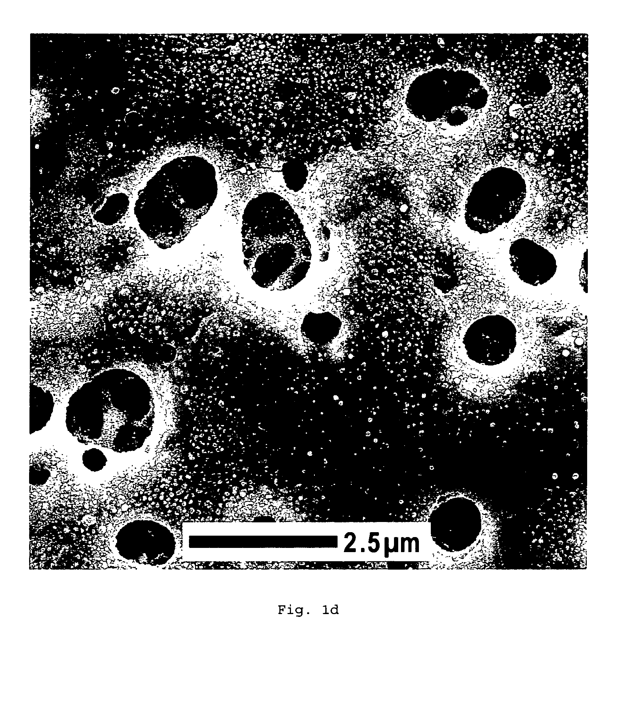

Fig. 1a zeigt ein REM-Bild einer eindimensionalen Kompositstruktur durch thermolytische Zersetzung von (tBuAlH₂)₂ auf Glassubstraten bei 600 °C. Die Nanodrähte der eindimensionalen Kompositstruktur haben einen gleichmäßigen Durchmesser von ca. 20-30 nm und bestehen aus einem inneren Kern aus Al mit einer Umhüllung aus Al₂O₃ im Verhältnis von Al/Al₂O₃=1:1 bei eine Länge von mehreren Mikrometern (Veith M, Sow E, Werner U, Petersen C, Aktas O. The Transformation of Core/Shell Aluminium/Alumina Nanoparticles into Nanowires. Eur J Inorg Chem 2008(33):5181-5184). Nach einem einzigen Laserpuls mit 0.2 J/cm³ an Luft ändert die Oberfläche am Punkt der Bestrahlung ihre Farbe (von schwarz nach weiß). Nach einem zweiten und einem dritten Laserpuls wird diese Farbänderung immer deutlicher. Die Figuren 1b-d zeigen die Oberflächenstruktur der Beschichtung nach Bestrahlung mit einem, zwei und drei Laserpulsen. Nach einem Laserpuls scheint die Oberfläche der Beschichtung als wäre sie geschmolzen und wieder erstarrt. Das erstarrte Material bedeckt die darunter liegenden Nanostrukturen (Fig. 1b). Bei dieser Oberflächenstruktur sind wenige runde Nanoerhebungen zu erkennen. Nach einem zweiten Laserpuls wird die Oberfläche relativ glatt (Fig. 1c), aber immer noch erinnern einige Strukturen an die darunterliegende eindimensionale Kompositstruktur. Nach einem oder mehreren weiteren Laserpulsen wird die Bildung von Mikropartikel auf der geschmolzenen und wiedererstarrten Schicht beobachtet (Fig. 1d). Die Größe dieser größeren Strukturen liegt im Bereich von 3 bis 5 µm. Die Beschichtung weist eine Mikrostruktur im Bereich von 3 bis 5 µm auf.

Wichtige Veränderungen zeigen sich bei der Messung der Kontaktwinkels mit Wasser als Messflüssigkeit (Fig. 1). Die Beschichtung vor der Bestrahlung zeigt einen Kontaktwinkel von ungefähr 10°. Dieser Wert steigt auf 43° an nach der Bestrahlung mit einem Laserpuls. Dies ist ebenfalls ein Hinweis auf die Ausbildung einer strukturierten Beschichtung mit Mikro- und Nanostrukturen. Bei der Bestrahlung mit einem zweiten oder dritten Laserpuls sinkt der Kontaktwinkel auf 36°, bzw. 14° ab.

Die quadratische Rauheit einer eindimensionalen Kompositstruktur vor der Bestrahlung liegt bei 8.53 +/-0.5 µm. Nach einem Laserpuls sinkt diese auf nur 4.22 +/-0.2 µm ab. Weitere Pulse lassen die Rauheit wieder auf 5.12 +/-0.3 µm, bzw. 6.37 +/-0.4 µm ansteigen. Dies zeigt ebenfalls die Bildung von Auswölbungen und Rillen auf der Oberfläche.

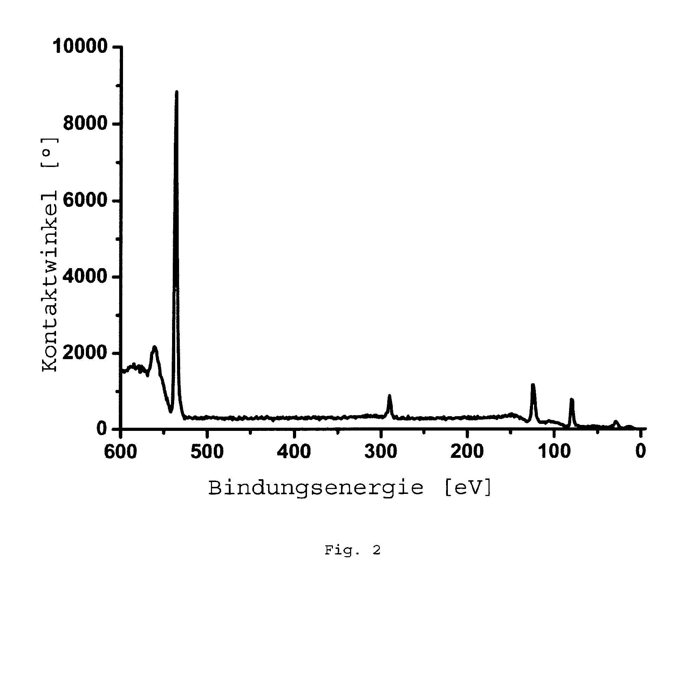

Ein XPS-Spektrum (Röntgen-Photoelektronenspektroskopie) einer Beschichtung, welche mit einem Laserpuls bestrahlt wurde, zeigt Figur 2. Die beobachteten Signale stammen von den Al2p, Al2s, Ols und C1s Elektronen. Zusätzlich sind noch O (KKL) Auger Signale zu erkennen. Die C1s-Signale stammen wahrscheinlich aus einer Verunreinigung während der Probenvorbereitung oder von einem Rest Pumpenöl. Der Kohlenstoffgehalt blieb bei allen Bedingungen (vor und nach der Bestrahlung) konstant bei 5%. Dies zeigt, dass sich nach der Laserbestrahlung keine Kohlenstoffhaltige Schicht bildet. Die chemische Zusammensetzung an der Oberfläche zeigt keine Veränderung durch die Laserbestrahlung. Dies war zu erwarten, da bei der Oxidation des Aluminiums Al₂O₃ gebildet wird, welches auch schon vorher die Oberfläche der Beschichtung bildete.

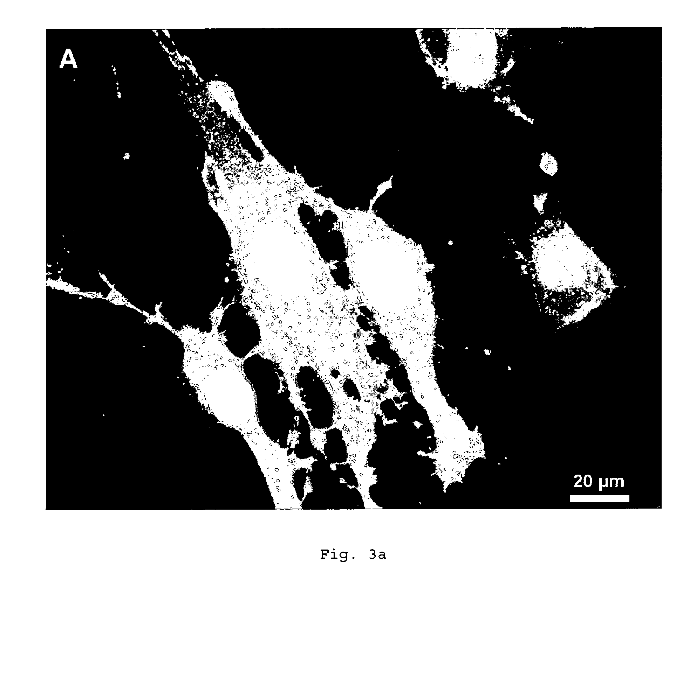

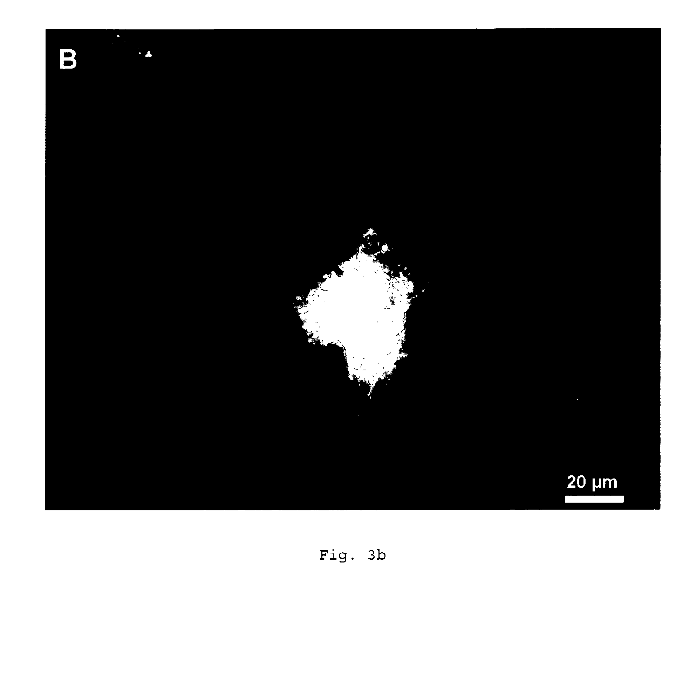

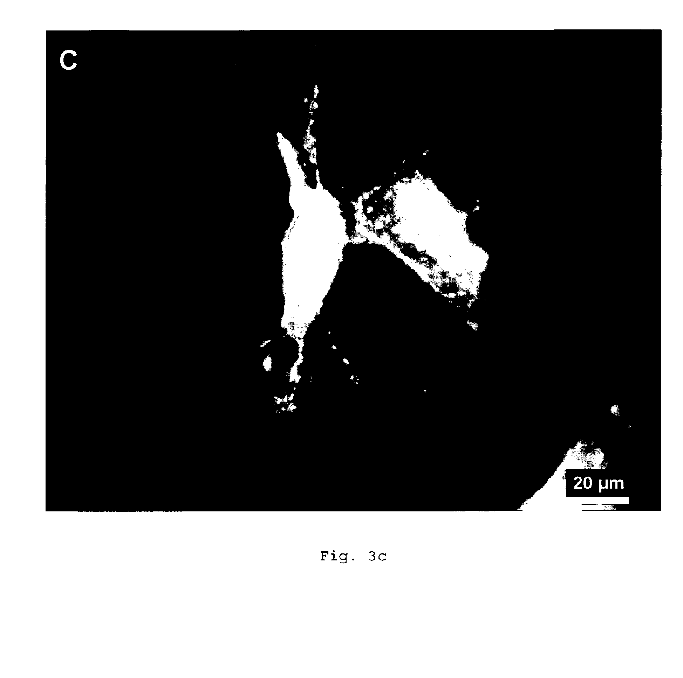

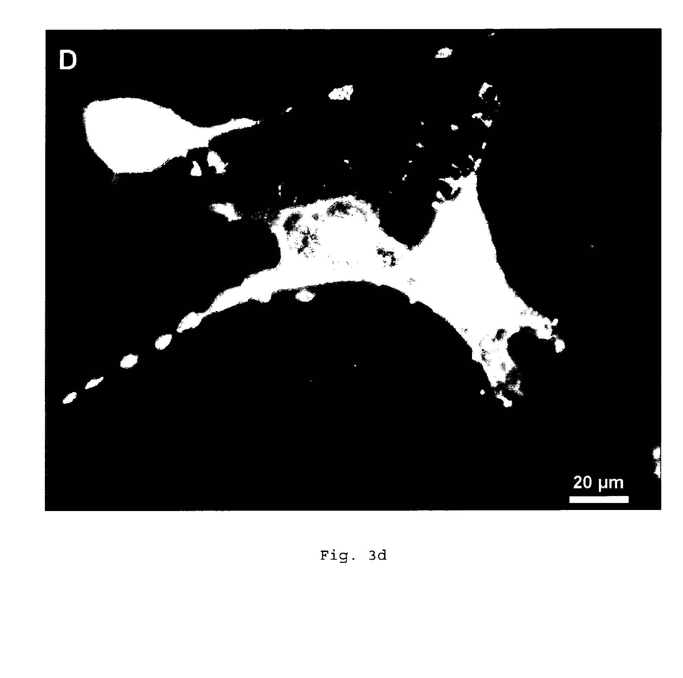

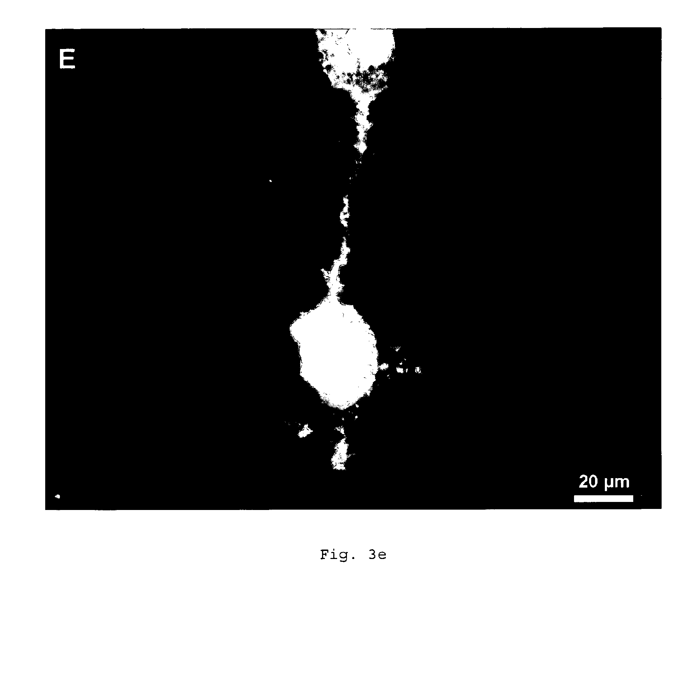

Fig. 3 zeigt typische beobachtete Zellmorphologien auf unterschiedlichen Proben. Fig. 3a zeigt NHDF als Kontrolle auf einer Glasplatte. In allen untersuchten Feldern (n=100) konnten 25 mitotische Zellen beobachtet werden. Die Zelldichte und die Anzahl der mitotischen Zellen sind dagegen auf allen beschichteten Substraten deutlich reduziert. So wurden auf der eindimensionalen Kompositstruktur und der mit einem Laserpuls behandelten Beschichtung 4 mitotische Zellen gefunden, aber keine mitotische Zellen auf mit mehr als einem Laserpuls behandelten Beschichtungen. Die Mehrheit der Zellen auf der eindimensionalen Kompositstruktur (nicht bestrahlt) zeigt eine ungewöhnliche Zellmorphologie mit einer deutlich reduzierten Zellgröße (Fig. 3b). Auf den mit einem oder zwei Laserpulsen behandelten Beschichtungen ähnelt die Morphologie der Zellen den Zellen auf dem Kontrollsubstrat Glas (Fig. 3c, bzw. 3d). Die Zellen auf den Beschichtungen, welche mit drei Laserpulsen behandelt wurden, sind dagegen deutlich verändert. Die Zellen sind sehr klein mit kleinem Zellkern und unter dem Mikroskop wegen ihrer geringen Größe und ihrer unregelmäßigem Morphologie kaum zu erkennen (Fig. 3e). Untersuchungen mit AAS ergaben, dass bei keinem der Versuche Al³⁺-Ionen im Zellkulturmedium zu finden waren.

Die Kontrollen auf Glas weisen schon schon bei bloßer mikroskopischer Betrachtung eine deutlich höhere Zelldichte aufzuweisen, als alle verwendeten beschichteten Substrate.

Die höchste Zelldichte wurde auf den Kontrollsubstraten aus Glas gefunden mit 96 Zellen pro Quadratmillimeter (Zellen/mm²). Verglichen mit der Anfangsdichte der Kultivierung von 63 Zellen/mm² ist dies ein deutlicher Anstieg und ein deutliches Zeichen für erfolgte Zellteilung. Auf allen anderen beschichteten Substraten ist die Zelldichte drastisch reduziert. Die geringsten Zelldichten von ca. 15 Zellen/mm² wurden auf der eindimensionalen Kompositstruktur ohne Bestrahlung und auf der mit drei Laserpulsen bestrahlten Beschichtung gemessen. Verglichen damit weisen die Beschichtungen, welche mit nur einem Laserpuls behandelt wurden, eine höhere Zelldichte auf.

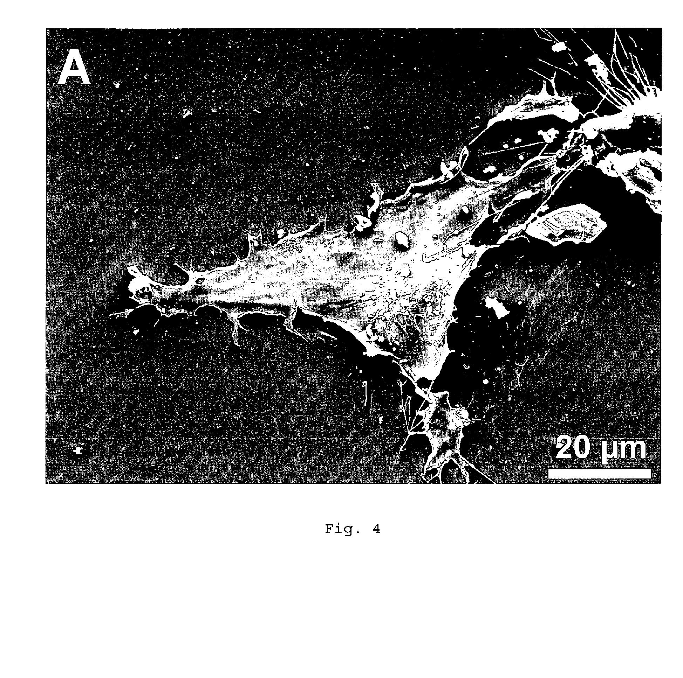

Über REM lassen sich auch die Adhäsion einzelner Zellen und die Topographie des Substrats gleichzeitig untersuchen. Die Zellen auf dem Glassubstrat bilden viele Filopodien um am Substrat zu haften (Fig. 4). Die meisten der Filopodien sind sehr verzweigt und zeigen einige ausgedehnte Bereiche und Verbreiterungen, um den Kontakt mit der Oberfläche zu erhöhen.

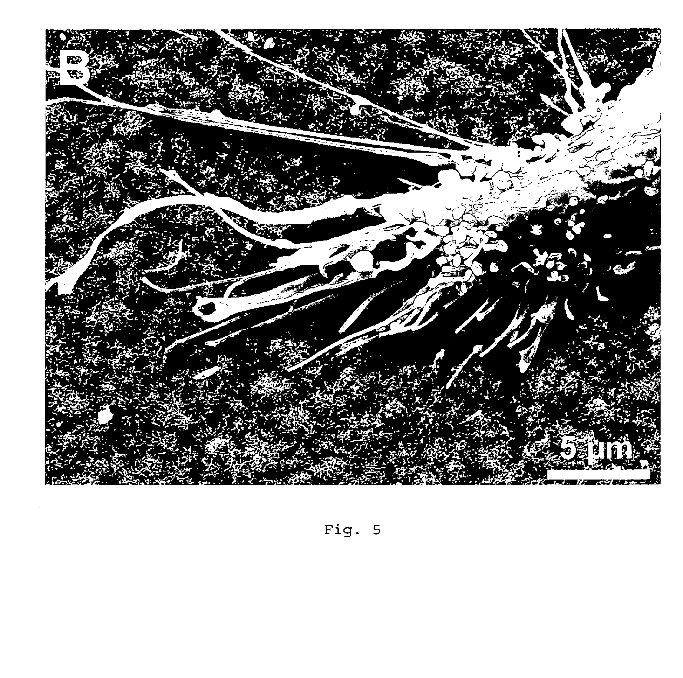

Auf der eindimensionalen Kompositstruktur ohne Bestrahlung zeigen die Zellen eine ziemlich kleine und ungewöhnliche Morphologie verglichen mit den Zellen auf der Glasoberfläche (Fig. 5). Nur wenige Filopodien sind zu erkennen und sie sind nicht verzweigt oder verbreitert. Einige Filopodien scheinen keinen Kontakt zur Oberfläche zu haben. Dies war in einigen Fällen auch direkt sichtbar, da die Filopodien sich während der Aufnahme mit dem REM bewegten. Zusätzlich zeigt Fig. 5 die Topographie der eindimensionalen Kompositstruktur ohne Bestrahlung, insbesondere mit einer Sekundärstruktur, welche an eine Himbeere erinnert.

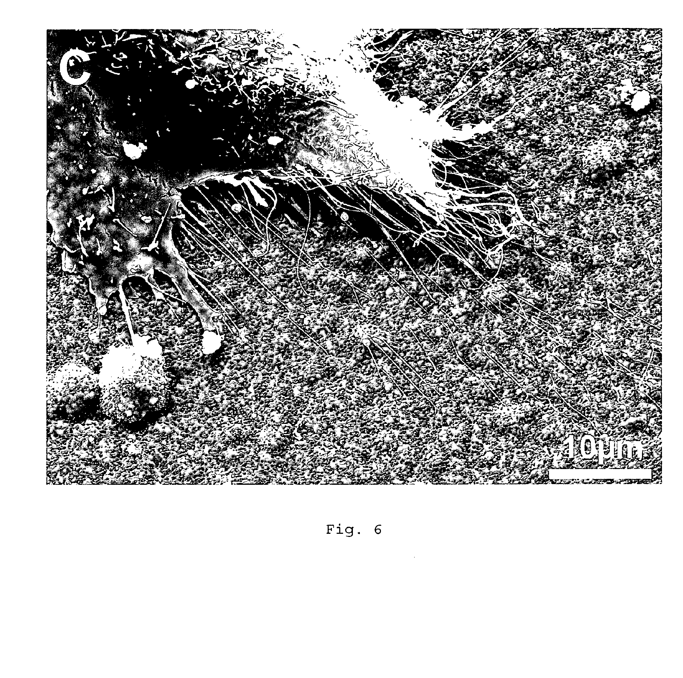

Die mit einem Laserpuls behandelten eindimensionalen Kompositstrukturen weisen eine völlig andere Topographie auf. Die meisten der Nanodrähte sind geschmolzen und die Himbeerförmige Sekundärstruktur ist verschwunden. In einigen Poren ist die Nanostruktur noch zu erkennen (Fig. 6). Die Morphologie der Zellen in Fig. 6 ähnelt der der Zellen in Fig. 4. Es sind viele Filopodien zu erkennen, welche - wenn auch im geringen Ausmaß - verzweigt sind und verbreiterte Bereich aufweisen. Die Filopodien scheinen die glatteren Bereiche der Oberfläche zur Anhaftung zu bevorzugen.

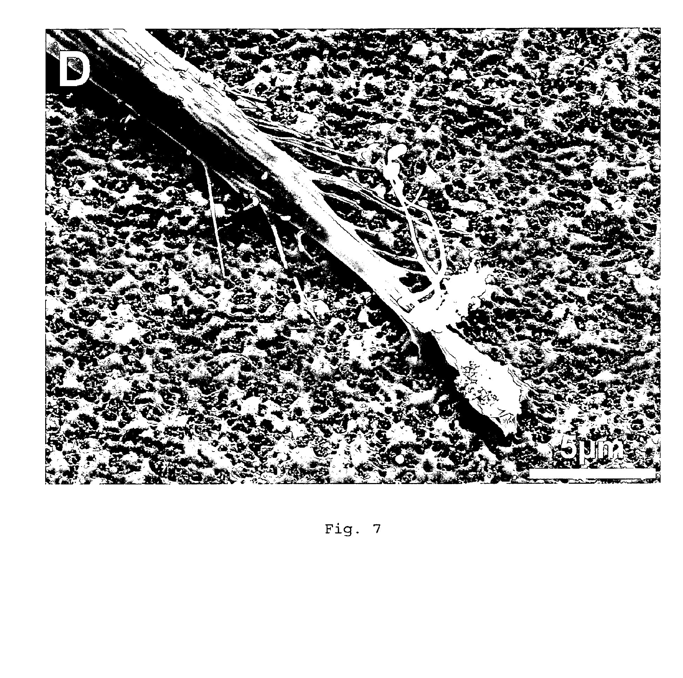

Die Behandlung mit zwei Laserpulsen verändert die Topographie der Oberfläche nur gering (Fig. 7). Die Zellen zeigen eine normale Morphologie und einige Filopodien sind zu erkennen. Die Zellen scheinen erneut die glatteren Bereiche der Oberfläche zur Anhaftung zu bevorzugen..

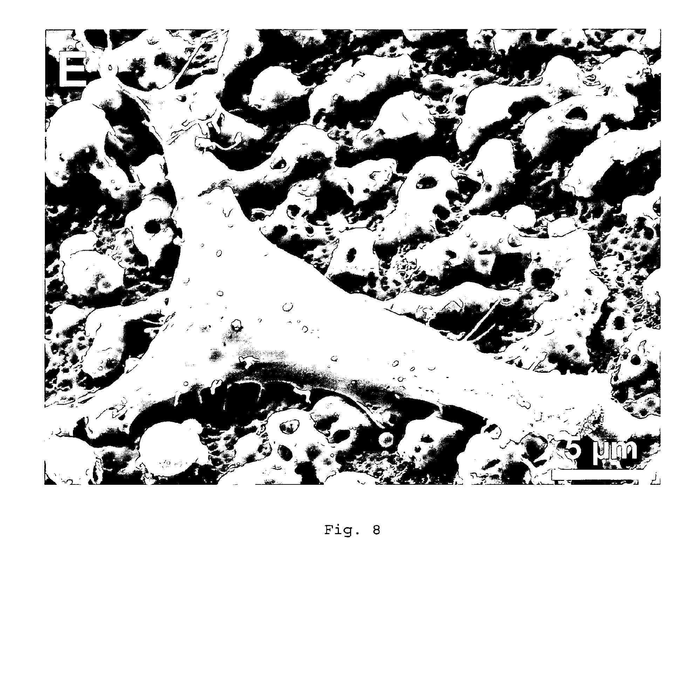

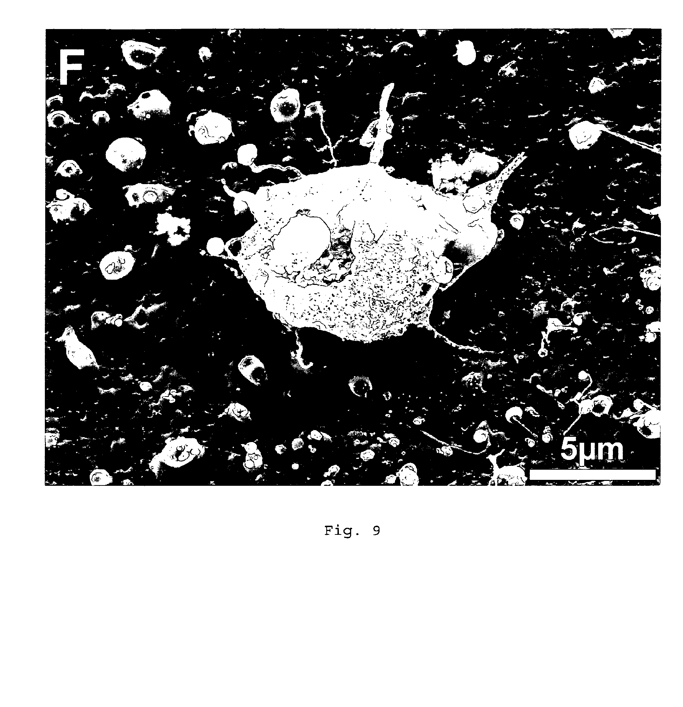

Nach der Behandlung mit drei Laserpulsen verändert sich die Topographie der Oberfläche sehr stark. Die gesamte Oberfläche übersät mit kugelförmigen Partikeln, welche vielleicht durch den Ablationsdruck als Folge des hohen Energieeintrags des Laserpulses gebildet werden (Fig. 8 und Fig. 9). Die auf dieser Beschichtung kultivierten Zellen sind in sehr schlechter Verfassung, was in Fig. 9 deutlich zu erkennen ist. Einige Zellen konnten Filopodien ausbilden und an den sphärischen Strukturen anhaften (Fig. 8). Dennoch bestätigen diese Aufnahmen die Resultate aus den Messungen der Zelldichte.

### Material und Methoden

Herstellung der eindimensionalen Kompositstruktur (WO 2008011920 A1; Veith M, Kneip S. New metal-ceramic comosites grown by metalorganic chemical-vapor-deposition. J Mater Sci Lett 1994;13(5):335-337; Veith M, Faber S, Hempelmann R, Janssen S, Prewo J, Eckerlebe H. Synthesis and microstructure of nanostructured Al/Al2O3(H)-composite. Journal of Materials Science 1996;31(8):2009-2017))

Al/Al₂O₃ Kompositstrukturen wurden durch thermolytische Zersetzung von (tBuOAlH₂)₂ auf ein Glassubstrat in einer CVD-Kammer. Das Verfahren ist in der Literatur beschrieben. Zusammengefasst wurde der Precursor durch Reaktion von AlCl₃ und LiAlH₄ in tert-Butanol erhalten. [25]. Das Substrat wurde auf 600 °C erhitzt und bei reduziertem Druck von 2 x 10⁻² mbar für 45 Minuten einem konstanten Strom des Precursors ausgesetzt. Danach wurden die Substrate unter Vakuum auf Raumtemperatur abgekühlt.

### Laserbehandlung der eindimensionalen Kompositstruktur

Die Proben wurden mit einem gepulsten Nd:YAG-Laser (Quanta-Ray PRO 290, Spectra Physics) behandelt. Die Wellenlänge betrug 532 nm bei einer Pulslänge von 8 ns und einer Pulsfrequenz von 10 Hz. Der Laser war horizontal polarisiert und traf horizontal ausgerichtet senkrecht auf die Oberfläche der Proben ohne eine Fokussierung. Die Anzahl der Pulse (P) wurde über einen schnellen mechanischen Verschluss geregelt. Die Fluenz des Lasers betrug 0.2 J/cm². Die Proben wurden mit 1 bis 3 Pulsen bestrahlt.

### Charakterisierung der Oberfläche

Die behandelten und unbehandelten Proben wurden unter einem SEM (FEI Quanta 400 FEG) bei einer Beschleunigungsspannung von 10 kV untersucht.

Die Zusammensetzung der Beschichtung wurde mit einem PHI 5600 XPS mit monochromatischen Al Kα Strahlen untersucht.

Der Kontaktwinkel wurde mit Hilfe eines Videosystems bestimmt. Die Proben wurden auf einem planen Tisch platziert und ein Tropfen destilliertes Wasser auf den Proben aufgebracht. Der Kontaktwinkel wurde aus dem Durchschnittswert von 4 Messungen an unterschiedlichen Stellen einer Probe ermittelt.

Die Rauheit der Oberfläche wurde mit einem Profilometer bei Raumtemperatur aufgenommen. Für jede Probe wurden 5 Profile an unterschiedlichen Stellen aufgenommen. Die quadratische Rauheit (rms-roughness = root-mean-squared roughness) als Durchschnittswert der quadratischen Rauheit der 5 Profile ermittelt.

### Zellkultur

Für die Zellkultur wurden soweit nicht anders angegeben übliche Standardprozeduren. Die Substrate wurden durch dreimaliges Spülen mit steriler Phosphat-gepufferten Salzlösung (PBS), Inkubation in 70%-Ethanol für 5 Minuten und anschließender zweimaliger Spülung mit PBS. Fibroblast-Zellen (NHDF, *Normal Human Dermal Fibroblasts,* Promocell) wurden in Quantum 333 (Q333, PMAlaboratories) in 75 cm² Zellkulturflaschen (Greiner BioOne) kultiviert bis Konfluenz erreicht war. Für die Experimente wurden nur Zellkulturen, welche weniger als 10mal transferiert worden waren. Die NHDF-Zellen wurden mit üblichen Verfahren vom Boden der Zellkulturflaschen gelöst. Die Inkubationsparameter waren 37 °C, 95 % Luftfeuchtigkeit und 5% CO₂. Die Zelldichte wurde mittels eines CASY^{®}-Zellzähler (Schärfe Systemtechnik) bestimmt. Die Substrate und Standard-Objektträger wurden mit einer anfänglichen Zelldichte von 63 Zellen/mm² angeimpft. Die Inkubation wurde in flachen Schalen (Quadriperm, Greiner BioOne) in 4 ml Q333 für 2 Tage durchgeführt. Ein Aliquot des Ursprungsmediums und der Mediums nach Inkubation wurden bei - 20°C aufbewahrt, zur Untersuchung auf Aluminiumionen. Die Al³⁺-Analyse wurde mittels Atomabsorptionsspektroskopie (AAS, Quanta) durchgeführt. Die Zellmorphologie und Zelldichte wurde täglich photographisch dokumentiert.

### Immunofluoreszenz-Markierung

Die NHDF wurden mit einem konstitutiven Membran-bindenden Marker für Fibroblasten (CD90) um ihre Morphologie durch Fluoreszenzspektroskopie zu bestimmen.

Am Ende der Inkubation wurde das Medium entfernt und die Zellen (und die Substrate) dreimal mit PBS gespült (37 °C) und anschließend für 5 Minuten bei 37 °C mit KCl-Lösung (0.05 M) behandelt. Die Zellen wurden durch Behandlung mit kaltem Methanol (-20 °C) für mindestens 10 Minuten fixiert. Danach wurden die Zellmembranen durch zweifache Inkubation mit PBS mit 0.05% Tween 20 für 5 Minuten permeabilisiert. Um unspezifische Bindung zu minimieren wurden die Substrate mit PBS mit 0.1 % BSA (*bovine serum albumin*) behandelt. Als Primärantikörper wurde ein Maus-Anti-Human-CD90 Antikörper (Dianova, Hamburg, 1:200 in PBS mit 0.1% BSA) zugegeben (75 µl). Die Proben wurden für 30 Minuten in einer dunklen feuchten Kammer bei Raumtemperatur inkubiert. Nach dreimaligem Waschen mit PBS mit 0.5 % Tween 20 wurde als Sekundärantikörper ein Ziege-Anti-Maus-Antikörper mit Cy3-Markierung zugegeben und ebenfalls wie vorstehend beschrieben inkubiert. Danach wurden die Proben dreimal mit PBS mit 0.5% Tween 20 gewaschen und die Markierung durch Inkubation mit 4% Paraformaldehyd in PBS für 5 Minuten fixiert und danach noch einmal mit PBS mit 0.5% Tween 20 gewaschen. Nach Dehydrierung der Proben durch mehrfache Behandlung mit Ethanol (70%, 80%, 96%) wurden die Proben unter Mikroskopiermedium (*mounting medi*um) mit 4'-6-diamidino-2-phenylindole (DAPI) für Zellkernmarkierung (Vectashield, Vector Laboratories) bei 4 °C gelagert, bis die mikroskopische Untersuchung durchgeführt wurde.

### Mikroskopische Analyse

Die mikroskopischen Analysen wurden mit einem Zeiss Axioskop Mikroskop und der Axiovision Software bei 400facher Vergrößerung durchgeführt. Die Zellen in 20 Feldern wurden gezählt und die absoluten Zahlen in Zellen pro Millimeter umgerechnet. Die erhaltenen Werte wurden durch invariante Varianzanalyse (*Oneway ANOVA for repeated measures*) auf ihre Signifikanz (p<0.05) hin untersucht.

### REM-Analyse der Zellen

NHDF wurden wie vorstehend beschrieben kultiviert. Um das Q333-Medium zu entfernen, wurden die Substrate zweimal mit PBS (37 °C) gespült und mit 1% Paraformaldehyd und 1% Glutaraldehyd in 0.12M PBS für 2 Stunden bei Raumtemperatur und unter Bewegung fixiert. Danach wurden die Proben mit Osmiumtetroxid (4% in desionisiertem Wasser dH₂O) für 2 Stunden unter Bewegung im Dunkeln inkubiert. Danach wurden die Proben über Nacht in dH₂O bei 4 °C aufbewahrt. Die Zellen wurden durch zweifaches Behandeln in einer Ethanol-Konzentrationsreihe (30%, 50%, 70%, 80% und 90%) bei 4 °C für 5 Minuten unter Bewegung getrocknet. Die Dehydrierung der Zellen wurde durch dreifaches Behandeln mit 100% Ethanol für 15 Minuten bei 4 °C unter Bewegung abgeschlossen. Die Proben wurden danach durch Kritischer-Punkt-Trocknung (*Critical-Point-Drying,* Polaron CPD 7501, Quorom Technologies) getrocknet und mit Gold-Palladium gesputtert (Polaron, Sputter Coater). Die Proben wurden in einem Rasterelektronenmikroskop (REM; *Scanning Electron Microscope SEM;* FEI XL 30 ESEM FEG SEM, Hilsboro) analysiert.

Liste der zitierten Literatur:
WO 2008011920 A1
Veith M, Faber S, Hempelmann R, Janssen S, Prewo J, Eckerlebe H. Synthesis and microstructure of nanostructured Al/Al2O3(H)-composite. Journal of Materials Science 1996;31(8):2009-2017
Veith M, Kneip S. New metal-ceramic comosites grown by metalorganic chemical-vapor-deposition. J Mater Sci Lett 1994;13(5):335-337.
Veith M, Sow E, Werner U, Petersen C, Aktas O. The Transformation of Core/Shell Aluminium/Alumina Nanoparticles into Nanowires. Eur J Inorg Chem 2008(33):5181-5184
Jager M, Zilkens C, Zanger K, Krauspe R. Significance of nanoand microtopography for cell-surface interactions in orthopaedic implants. J Biomed Biotechnol 2007;2007(8):69036.
Hulbert SF, Young FA, Mathews RS, Klawitter JJ, Talbert CD, Stelling FH. Potential of ceramic materials as permanently implantable skeletal prostheses. J Biomed Mater Res 1970;4(3):433-456.
Webster T, Hellenmeyer E, Price R. Increased osteoblast functions on theta plus delta nanofiber alumina. Biomaterials 2005;26(9):953-960.
Swan E, Popat K, Grimes C, Desai T. Fabrication and evaluation of nanoporous alumina membranes for osteoblast culture. Journal of Biomedical Materials Research Part A 2005;72A(3):288-295.

## Patentansprüche

1. Verwendung einer strukturierten Beschichtung zur Beschichtung von Implantaten, wobei die strukturierte Beschichtung mindestens aus einem Oxid ausgewählt aus Aluminiumoxid, Galliumoxid, Indiumoxid und Thalliumoxid besteht und eine Mikrostruktur und/oder eine Nanostruktur aufweist und die Beschichtung durch Bestrahlung einer eindimensionalen Kompositstruktur erhalten wurde.

2. Verwendung einer strukturierten Beschichtung nach Anspruch 1, **dadurch gekennzeichnet, dass**
die Beschichtung eine Strukturierung im Bereich von 1 bis 10 µm aufweist.

3. Verwendung einer strukturierten Beschichtung nach Anspruch 1, **dadurch gekennzeichnet, dass**
die Beschichtung eine eindimensionale Kompositstruktur umfasst.

4. Verwendung einer strukturierten Beschichtung nach einem der Ansprüche 1 bis 3, **dadurch gekennzeichnet, dass**
die Beschichtung einen Kontaktwinkel von unter 40°, bevorzugt unter 20° mit Wasser als Messflüssigkeit aufweist.

5. Verwendung einer strukturierten Beschichtung nach einem der Ansprüche 1 bis 4, **dadurch gekennzeichnet, dass**
die Beschichtung eine quadratische Rauheit von über 5 µm aufweist.

## Claims

1. Use of a structured coating for the coating of implants, wherein the structured coating consists of at least one oxide selected from aluminum oxide, gallium oxide, indium oxide and thallium oxide and has a microstructure and/or a nanostructure and wherein the coating was obtained by irradiation of a one-dimensional composite structure.

2. The use of a structured coating as claimed in claim 1, **characterized in that** the coating exhibits a structuring in the range from 1 to 10 µm.

3. The use of a structured coating as claimed in claim 1, **characterized in that** the coating comprises a one-dimensional composite structure.

4. The use of a structured coating as claimed in one of claims 1 to 3, **characterized in that** the coating exhibits a contact angle of less than 40°, preferably less than 20°, with water as the measurement liquid.

5. The use of a structured coating as claimed in one of claims 1 to 4, **characterized in that** the coating exhibits a quadratic roughness of greater than 5 µm.

## Revendications

1. Utilisation d'un revêtement structuré pour le revêtement d'implants, le revêtement structuré étant constitué d'au moins un oxyde choisi parmi l'oxyde d'aluminium, l'oxyde de gallium, l'oxyde d'indium et l'oxyde de thallium et présentant une microstructure et/ou une nanostructure, et le revêtement ayant été obtenu par exposition à un rayonnement d'une structure composite monodimensionnelle.

2. Utilisation d'un revêtement structuré selon la revendication 1, **caractérisée en ce que** le revêtement présente une structuration dans la plage allant de 1 à 10 µm.

3. Utilisation d'un revêtement structuré selon la revendication 1, **caractérisée en ce que** le revêtement comprend une structure composite monodimensionnelle.

4. Utilisation d'un revêtement structuré selon l'une quelconque des revendications 1 à 3, **caractérisée en ce que** le revêtement présente un angle de contact de moins de 40°, de préférence de moins de 20°, avec l'eau en tant que fluide de mesure.

5. Utilisation d'un revêtement structuré selon l'une quelconque des revendications 1 à 4, **caractérisée en ce que** le revêtement présente une rugosité quadratique de plus de 5 µm.
